# FASCICULE DE BREVET EUROPEEN

(11) **EP 0 527 061 B1**
(45) Date de publication et mention de la délivrance du brevet: **10.05.2000**
(21) Numéro de dépôt: 92401484.8
(22) Date de dépôt: 01.06.1992
(51) Int. Cl.: C08B 37/00

(54) **Composition dérivant d'un succinoglycane, son procédé de préparation et ses applications**
Succinoglycan enthaltende Zusammensetzung, Verfahren zu ihrer Herstellung und ihre Anwendungen
Composition derived from a succinoglycan, process for obtaining the same and its uses

(30) Priorité: 10.07.1991 FR 9108670
(43) Date de publication de la demande: 10.02.1993
(73) Titulaire: RHODIA CHIMIE, 92408 Courbevoie Cédex (FR)
(72) Inventeur: Knipper, Magali, F-75019 Paris (FR); Senechal, Alain, F-94700 Maison-Alfort (FR); Raffart, Michèle, F-75012 Paris (FR)
(74) Mandataire: Seugnet, Jean Louis

(56) Documents cités:
- EP-A- 0 215 692
- EP-A- 0 237 418
- GB-A- 2 176 795

## Description

La présente invention se rapporte à une nouvelle composition dérivant d'un succinoglycane.

Par le terme succinoglycane on désigne généralement une famille de polysaccharides d'origine bactérienne qui comportent outre des motifs dérivés du galactose et du glucose, des motifs dérivés des acides succinique, pyruvique et éventuellement acétique ou des sels de ces acides.

Les différents microorganismes qui sont susceptibles de conduire à ces succinoglycanes sont en particulier les bactéries du type Pseudomonas, Rhizobium, Alcaligenes et Agrobacterium.

Quelques succinoglycanes particuliers ont déjà été décrits dans la littérature pour leurs propriétés rhéologiques performantes dans des conditions de températures ou de pH drastiques.

Ces succinoglycanes présentent en effet, des propriétés rhéologiques comparables à celles de la gomme xanthane et sont en outre dotés d'une stabilité accrue, à la température, aux pH acides et basiques et dans des milieux fortement salins.

Ce type de succinoglycanes s'avère donc un suppléant avantageux de la gomme xanthane dans des domaines industriels aussi variés que par exemple l'agrochimie, l'agro-alimentaire, l'industrie pétrolière et la cosmétique.

Diverses applications industrielles des succinoglycanes ont déjà été décrites. Toutefois, ces applications mettent en oeuvre uniquement des solutions aqueuses de succinoglycane et non des gels aqueux de succinoglycane. Or, ce type de gel serait tout particulièrement intéressant pour ses capacités supérieures à titre d'agent suspensif et stabilisant tant en alimentaire qu'en cosmétique.

Des gels de succinoglycanes sont décrits dans le brevet EP 251 638 mais il s'agit essentiellement de gels obtenus via la réticulation des succinoglycanes par des cations métalliques. Ces cations métalliques demeurent dans le gel de succinoglycane final qui s'avère donc incompatible avec des applications en industrie alimentaire.

La présente invention a précisément pour objet une nouvelle composition dérivant d'un succinoglycane et qui possède plus particulièrement la faculté de conduire à des gels stables, dotés d'une parfaite innocuité et donc compatibles avec une utilisation en industries alimentaire et cosmétique.

La présente invention a également trait à un procédé de préparation de cette composition.

Enfin, un troisième objet de l'invention vise les applications de ladite composition.

Plus précisément la présente invention se rapporte à une composition à base d'au moins un polysaccharide caractérisée en ce qu'elle dérive d'au moins un succinoglycane et en ce que ledit polysaccharide présente une teneur réduite en motifs dérivés des acides succinique et pyruvique ou de leurs sels par rapport au succinoglycane d'origine.

De manière générale, la teneur du polysaccharide est réduite respectivement d'au moins 30% en motifs dérivés de l'acide pyruvique et d'au moins 50% en motifs succiniques par rapport au succinoglycane d'origine. Plus particulièrement, la teneur du polysaccharide en motifs pyruviques est de préférence réduite environ de 30 à 80% et en motifs succiniques environ de 50 à 80% par rapport au succinoglycane d'origine.

La composition selon l'invention dérive d'un succinoglycane issu de la fermentation d'une souche de carbone assimilable par un microorganisme de la souche Agrobacterium tumefaciens I-736, un de ses recombinants ou un de ses mutants.

La composition selon l'invention peut se présenter indifféremment sous la forme d'un gel aqueux ou sous la forme d'un solide de type fibres ou poudre, gélifiable en milieu aqueux.

Dans ce dernier cas le gel est obtenu par simple dispersion de la composition sous une forme pulvérulente dans un solvant aqueux.

La concentration de ce gel, objet de l'invention, en polysaccharide varie généralement entre environ 0,1 à environ 5%, de préférence entre environ 0,2 et 1% et plus particulièrement est proche de 0,3%.

La force de ce gel est bien entendu modulable selon la concentration et la nature du succinoglycane d'origine mis en oeuvre.

Ce gel est stable dans une large gamme de pH et son pH peut être ajusté à la valeur souhaitée par simple ajout d'une base ou d'un acide convenable.

Dans le cas particulier d'une composition selon l'invention dérivant d'un succinoglycane issu de la souche spécifique Agrobacterium tumefaciens I-736 le gel correspondant est un gel transparent.

L'analyse chimique d'un gel issu de cette souche spécifique montre que pour un galactose on trouve environ 8,6 glucoses et seulement environ 0.26 succinique et environ 0.32 pyruvique contre 0,8 succinique et 1 pyruvique pour le succinoglycane d'origine.

La composition selon l'invention est susceptible d'être obtenue par simple hydrolyse acide ou enzymatique d'au moins un succinoglycane.

Plus précisément, la présente invention se rapporte également à un procédé de préparation de ladite composition caractérisé en ce qu'il comprend:
- l'acidification à un pH au moins inférieur à 1,5 d'une solution aqueuse d'un succinoglycane d'origine,
- la conservation, éventuellement sous agitation, de cette solution acidifiée jusqu'à ce qu'elle atteigne un seuil de viscosité minimale puis forme un gel et
- la récupération de la composition.

De préférence, l'acidification est réalisée avec une solution aqueuse concentrée d'un acide minéral.

A titre d'acide minéral on peut plus particulièrement cité les acides chlorhydrique, sulfurique, nitrique et phosphorique. De préférence, il s'agit d'une solution aqueuse à 20% en acide sulfurique.

Des acides organiques peuvent également être utilisés mais il est clair qu'il est plus difficile d'atteindre avec ces derniers des pH très acides.

De préférence, l'acide est ajouté à une solution aqueuse à environ 0,3% en succinoglycane.

Suite à l'acidification, la formation d'une composition selon l'invention passe toujours, au préalable, par une chute complète de la viscosité de la solution et, le cas échéant, la formation d'un précipité. La composition ne se forme qu'ultérieurement sous la forme d'un gel.

La cinétique de formation du gel est liée à la force de l'acide utilisé, à sa concentration et à la température de stockage des solutions acidifiées de succinoglycane.

Cette solution acide peut être stockée à une température comprise entre environ 20 et 80°C. Toutefois un stockage à une température supérieure à 40°C et inférieure à 60°C accélère considérablement le phénomène de gélification. De préférence la solution est conservée à une température proche de 50°C.

Le gel ainsi obtenu est stable à la température ambiante. Son pH acide peut être réajusté au moyen d'une solution aqueuse basique convenable jusqu'à sa neutralité. Quant à la force du gel, elle est également bien entendu modulable selon la nature et la concentration de l'acide utilisé.

Les données figurant dans les exemples çi-après mettent en évidence les influences de ces différents paramètres, température, nature et force de l'acide sur la cinétique de la formation et la force du gel. Il est clair que l'homme de l'art est à même, à partir de ces données, d'obtenir par des expériences de routine le gel désiré.

Bien entendu, il est tout à fait possible d'envisager l'emploi de ce gel acide directement à titre de gel détartrant acide.

L'ajout de colorants et/ou de parfums ordinairement présents dans ce type de composition détergente peut se faire préalablement à la gélification de la solution de succinoglycane acidifiée c'est à dire au moment où sa viscosité atteint un seuil minimale.

Selon un mode de réalisation particulier de l'invention le procédé de préparation comprend en outre, lorsque le seuil de viscosité minimale est atteint et avant la formation du gel susbséquent, une étape de précipitation de ladite solution à l'aide d'un solvant organique, la récupération des fibres résultantes et éventuellement la purification de celles-çi.

A titre de liquides organiques convenables selon la présente invention on peut citer l'acétone, les alcools tels que l'éthanol, le propanol, l'isopropanol, le butanol, le tertio-butanol. L'isopropanol est plus particulièrement préféré dans le cadre de la présente invention.

Le volume de liquide organique utilisé est généralement 2 à 3 fois celui du volume de polysaccharide à traiter.

La précipitation par un liquide organique peut également être réalisée en présence de sels tels que les sulfates, chlorures ou phosphates de sodium, de potassium, ou de calcium.

Cette précipitation est de préférence réalisée à la température de conservation de la solution acide et par ajout de cette solution de polysaccharide acide au liquide organique. L'hétéropolysaccharide une fois précipité, peut ensuite être séparé du liquide organique par filtration, centrifugation ou essorage. Les fibres résultantes ainsi isolées, peuvent être déshydratées par exemple au moyen d'un alcool ou de l'acétone. Elles peuvent ensuite être séchées et broyées pour conduire à une composition selon l'invention sous forme de poudre ou encore remises en suspension en milieu aqueux pour obtenir une composition selon l'invention sous forme d'un gel.

Le succinoglycane d'origine est préparé classiquement par culture de la souche choisie sur un milieu de fermentation approprié et dans des conditions de températures adéquates.

En ce qui concerne plus particulièrement la préparation d'un succinoglycane à partir de la souche Agrobacterium tumefaciens I-736 elle a déjà été discutée de manière détaillée dans le brevet EP 351 303. La Demanderesse se contentera de rappeler çi-après certains points de ce procédé de préparation.

Le succinoglycane correspondant est obtenu par culture de cette souche en présence d'une source de carbone assimilable de type glucose, saccharose ou hydrolysats d'amidon, d'une source organique azotée comme la caséine, les caséinates, les farines de végétaux, les extraits de levure ou le corn steap liquor (CSL), de sels minéraux tels les sulfates métalliques, phosphates ou carbonates et, éventuellement d'oligoéléments.

Bien entendu, les différentes sources de carbone, d'azote et d'acides minéraux précisées çi-dessus sont fournies sans limitation de la portée de l'invention à ces composés spécifiques.

De manière générale cette fermentation est réalisée à des pressions comprises entre 1 et 4 bars à une température comprise entre 25 et 35°C et dans des conditions aérobies submergées. Le pH du milieu de fermentation est d'ordinaire compris entre 5 et 9 et de préférence entre 6 et 8 et peut être ajusté au cours de la fermentation.

Le succinoglycane est ensuite isolé du moût de fermentation en réalisant successivement un traitement thermique du moût à un pH compris entre 6 et 8 puis une précipitation du succinoglycane au moyen d'un solvant organique et de préférence d'isopropanol. Une fois séparé du moût, le succinoglycane est filtré, centrifugé, essoré et sèché. Les fibres obtenues sont éventuellement broyées avant d'être utilisées pour la préparation d'une composition selon l'invention.

La composition du type gel selon l'invention peut être appliquée avantageusement dans de nombreux domaines industriels.

De part sa formulation originale gélifiée, elle est tout particulièrement intéressante en tant qu'agent épaississant, agent de suspension ou en tant qu'agent stabilisant de dispersions.

Du fait de sa parfaite innocuité, elle peut être utilisée dans le domaine alimentaire, dans des formulations cosmétiques et pharmaceutiques. Elle est bien entendu également appropriée pour des applications à caractère plus industriel, dans l'industrie des explosifs par exemple et pour la formulation de compositions détergentes type nettoyants ménagers ou industriels et de compositions lubrifiantes.

Agrobacterium tumefaciens a été déposée conformément au Traité de Budapest, auprès de la Collection Nationale de Culture des Microorganismes (CNCM), le 1^{er} Mars 1988, où elle est publiquement accessible sous le N°I-736. Cette souche provient de la Collection Nationale de Bactéries Phytopathogène et est répertoriée sous le numéro CNBP 291 dans le catalogue 1974 de l'organisme curateur.

Les exemples présentés ci-après permettront de mettre en évidence d'autres avantages et caractéristiques de l'invention sans toutefois en limiter la portée. De façon générale et sauf indications contraires, les pourcentages et parties figurant çi-après sont définis en poids.

### EXEMPLE 1 : Procédé de préparation du succinoglycane d'origine

On fait fermenter par une souche Agrobacterium tumefaciens I-736 un milieu renfermant (en g/l) :

| | |
|---|---|
| CSL (corn steap liquor) | 11 |
| K₂HPO₄ | 4 |
| MgSO₄, 7H₂O | 0,5 |
| Saccharose | 25 |
| Eau potable qsp | 1 L |

Ce milieu est fermenté par ladite souche à une température de 28°C, dans une cuve de 20 litres BIOLAFFITE® contenant un volume utile de 15 litres.

Le milieu est soumis à une agitation de 400 tours/min obtenue au moyen de mobiles de type RUSHTON®.

Le milieu est aéré sous un débit d'air de 825 l/h.

Après 90 heures de fermentation, ce qui correspond à la consommation totale ou quasi totale du saccharose, on obtient 66 % en poids d'hétéropolysaccharide défini par rapport au poids de saccharose mis en oeuvre.

La viscosité du moût de fermentation, mesurée au moyen d'un viscosimètre Brookfield LVT ® avec une aiguille cylindrique 4, à 30 tours/min est de 6.800 mPa.s.

La récupération de l'hétéropolysaccharide est effectuée à partir de 2 Kg de ce moût qui sont traités thermiquement à 90°C pendant 30 min.

Au moût ainsi traité sont ajoutés 2300 ml d'alcool isopropylique (IPA). La précipitation est effectuée en présence de 150 g de sulfate de sodium. Les fibres issues de la précipitation sont ensuite déshydratées 2 fois en présence de 1200 ml d'IPA. Les fibres sont alors essorées, dilacérées et séchées dans une étuve à 85°C. La matière sèche récoltée est broyée et tamisée. On obtient alors une poudre de succinoglycane de couleur crème qui est directement utilisée pour préparer le gel selon l'invention.

### EXEMPLE 2 : Procédé de préparation d'une composition selon l'invention par hydrolyse acide d'un succinoglycane.

2,2 g de poudre obtenue selon l'exemple 1 sont au préalable hydratés dans 217,8 g d'eau. La poudre est ajoutée dans l'eau qui est agitée au moyen d'une pale défloculeuse qui tourne à la vitesse de 600 tr.min. pendant 20 minutes. Cette solution est ensuite maintenue au repos pendant 4 heures. On prélève 220 g d'une solution d'acide chlorhydrique à 20 % que l'on ajoute sous agitation aux 220 g de la solution hydratée précédente. Ce mélange est versé dans un récipient hermétique qui est stocké dans une étuve maintenue à une température de 50°C. Cette étuve est munie d'un système d'agitation par retournement et qui permet une répartition homogène de la température au sein de la solution. Au bout de 48 heures, le mélange est sorti de l'étuve en vu de sa précipitation. On mesure 900 ml d'alcool isopropylique que l'on verse dans un bécher muni d'une pale défloculeuse. Le bécher est trempé dans un bain-marie à 50°C de façon à maintenir l'alcool à une température comprise entre 40 et 50 °C. On verse goutte à goutte le mélange chaud et acide à base de polysaccharide dans l'alcool chaud sous agitation à 600 tr.min. Des fibres courtes et gonflées apparaissent au sein du mélange. La suspension de fibres est ôtée du bain-marie et l'agitation est maintenue pendant 20 minutes. La suspension de fibres est filtrée sur un büchner muni d'un papier filtre. Les fibres sont repulpées dans un bécher contenant 400 ml d'alcool isopropylique sous agitation. Les fibres sont séchées à basse température.

Caractérisation : Par analyse chimique des fibres on trouve pour un galactose 8,6 glucoses, contre seulement 0,26 succinique et 0,32 pyruvique. Par simple dissolution dans l'eau et pour des concentrations comprises entre 0,1 et 1 % le produit forme un gel translucide.

### EXEMPLE 3 : Variation des paramètres de préparation de la composition.

La préparation décrite en exemple 2 a été repétée à différentes températures pour diverses concentrations en HCl. Le tableau I ci-après rend compte des résultats obtenus.

**TABLEAU I**

| Température | ACIDE % | VISCOSITES EN mPa.S | | | |
|---|---|---|---|---|---|
| | | T.4H | T.7J | T.30J | T.90J |
| 20°C | 1 | 380 | 500 | 408 | 340 |
| | 3 | 368 | 462 | 412 | |
| | 10 | 408 | 400 | 362 | 220 |
| | | | | | GEL+TARD |
| 40°C | 1 | 464 | 336 | 320 | 238 |
| | 3 | 434 | 342 | 264 | 146 |
| | 10 | 368 | 280 | 80 | GEL |
| | | | | GEL | |
| | | | | 60J | |
| 50°C | 1 | 492 | 328 | 210 | 72 |
| | 3 | 460 | 282 | 94 | GEL |
| | | | | | 60J |
| | 10 | 358 | 114 | GEL | |
| | | | | 15J | |

### EXEMPLE 4 : Influence de la nature de l'acide sur la cinétique de formation d'une composition selon l'invention.

Des gels de polysaccharides ont été préparés conformément au procédé de l'exemple 2 en utilisant à titre d'acide, de l'acide sulfurique, ou phosphorique.

Les résultats obtenus sont présentés en tableaux 2 et 3.Ils montrent que la formation d'un gel est également observée avec l'acide sulfurique et l'acide phosphorique.

**TABLEAU II**

| CONCENTRATION EN ACIDE (%) H₂SO₄ | TEMPERATURE (°C) DE STOCKAGE | TEMPS (JOURS) POUR FORMATION DE GELS |
|---|---|---|
| 20 | 20 - 25 | 60 |
| 20 | 40 | 15 - 30 |
| 20 | 50 | 4 |
| 20 | 60 | 1 |
| 10 | 40 | 30 |
| 10 | 50 | 15 |

**TABLEAU III**

| CONCENTRATION EN ACIDE (%) PHOSPHORIQUE | TEMPERATURE (°C) DE STOCKAGE | TEMPS (JOURS) POUR FORMATION DE GELS |
|---|---|---|
| 20 | 40 | 80 |
| 20 | 50 | 60 |
| 20 | 60 | - |

### EXEMPLE 5 : Détermination de la force des gels

La force des gels qui s'évalue par la résistance du gel en fonction de la pénétration est déterminée à l'aide du pénétromètre STEVENS ® avec une vitesse de pénétration égale à 0,5mm/s.

Les deux gels testés ont été préparés avec une température d'incubation de 50°C, l'un avec une solution d'acide chlorhydrique de 10 % et l'autre avec une solution d'acide sulfurique à 20 %.

Dans les deux cas le gel testé dérive du succinoglycane issu de la souche spécifique Agrobactérium tumefaciens I-736. Les résultats sont présentés dans le tableau IV.

**TABLEAU IV**

| FORCE à LA RUPTURE | CONCENTRATION EN POLYSACCHARIDE | |
|---|---|---|
| 0,96 N | HCL | 1% |
| 1,53 N | H₂SO₄ | 0,3% |

## Revendications

1. Composition à base d'au moins un dérivé de succinoglycane, caractérisée en ce que le succinoglycane d'origine est obtenu à partir de la souche *Agrobacterium tumefaciens I-736*, et en ce que ledit dérivé présente une teneur réduite d'au moins 30 % en motifs dérivés de l'acide pyruvique et une teneur réduite d'au moins 50 % (50 % étant exclu) en motifs dérivés de l'acide succinique, par rapport au succinoglycane d'origine, et en ce que ladite composition se trouve sous forme de gel aqueux, ou d'un solide du type fibres ou poudres, gélifiable en milieu aqueux.

2. Composition selon la revendication 1, caractérisée en ce que la teneur du polysaccharide en motifs pyruviques est réduite de 30 à 80 %, et en motifs succinique de strictement supérieure à 50 % à 80 %, par rapport au succinoglycane d'origine.

3. Composition selon la revendication 1, caractérisé en ce que la concentration du gel en polysaccharide varie entre 0,1 à 5 %.

4. Procédé de préparation d'une composition telle que définie à l'une quelconque des revendications 1 à 3 sous forme de gel aqueux, caractérisé en ce qu'il comprend :
- l'acidification avec une solution aqueuse d'un acide minéral, à l'exception de l'acide nitrique, à un pH au moins inférieur à 1,5, d'une solution aqueuse d'un succinoglycane obtenu à partir de la souche *Agrobacterium tumefaciens I-736*,
- la conservation, éventuellement sous agitation, de cette solution acidifiée à une température supérieure à 40°C, jusqu'à ce qu'elle atteigne un seuil de viscosité minimale puis forme un gel, et
- la récupération de la composition.

5. Procédé selon la revendication 4, caractérisé en ce que l'acide minéral est choisi parmi les acides chlorhydrique, sulfurique, et phosphorique.

6. Procédé selon l'une des revendications 4 ou 5, caractérisé en ce que la solution aqueuse d'acide minéral est une solution d'acide à 20 %.

7. Procédé selon l'une quelconque des revendications 4 à 6, caractérisé en ce que la conservation de la solution acidifiée se fait à une température comprise entre 40 et 60°C.

8. Composition telle que définie à l'une quelconque des revendication 1 à 3, susceptible d'être obtenue par un procédé tel que défini à l'une quelconque des revendications 4 à 7.

9. Application d'une composition selon l'une quelconque des revendications 1 à 3 et 8, ou obtenue selon le procédé décrit à l'une quelconque des revendications 4 à 7, à titre d'agent de suspension, d'agent épaississant et/ou d'agent stabilisant de dispersion.

10. Application d'une composition selon l'une quelconque des revendications 1 à 3 et 8, ou obtenue selon le procédé décrit à l'une quelconque des revendications 4 à 7, dans les industries pétrolières, agrochimique, alimentaire, cosmétique, des explosifs, ainsi que dans les compositions lubrifiantes, les nettoyants ménagers ou industriels.

11. Composition agrochimique, alimentaire, cosmétique, explosive, lubrifiante, nettoyante ménager ou industriel, comprenant une composition selon l'une quelconque des revendications 1 à 3 et 8, ou obtenue selon le procédé décrit à l'une quelconque des revendications 4 à 7.

## Claims

1. Composition based on at least one succinoglycan derivative, characterized in that the starting succinoglycan is obtained from the strain *Agrobacterium tumefaciens I-736* and in that the said derivative exhibits, with respect to the starting succinoglycan, a content of units derived from pyruvic acid which is reduced by at least 30% and a content of units derived from succinic acid which is reduced by at least 50% (50% being excluded) and in that the said composition exists in the form of an aqueous gel or of a solid of the fibre or powder type which can form a gel in an aqueous medium.

2. Composition according to claim 1, characterized in that the content of pyruvic units in the polysaccharide is reduced by 30 to 80% and of succinic units by strictly greater than 50% to 80%, with respect to the starting succinoglycan.

3. Composition according to claim 1, characterized in that the concentration of polysaccharide in the gel varies between 0.1 and 5%.

4. Process for the preparation of a composition as defined in any one of claims 1 to 3 in the form of an aqueous gel, characterized in that it comprises:
- the acidification with an aqueous solution of an inorganic acid, with the exception of nitric acid, to a pH at least of less than 1.5, of an aqueous solution of a succinoglycan obtained from the strain *Agrobacterium tumefaciens I-736*,
- the retention, optionally with stirring, of this acidified solution at a temperature of greater than 40°C, until it reaches a minimum viscosity threshold and then forms a gel, and
- the recovery of the composition.

5. Process according to claim 4, characterized in that the inorganic acid is chosen from hydrochloric acid, sulphuric acid and phosphoric acid.

6. Process according to either of claims 4 and 5, characterized in that the aqueous inorganic acid solution is a 20% acid solution.

7. Process according to any one of claims 4 to 6, characterized in that the retention of the acidified solution is carried out at a temperature of between 40 and 60°C.

8. Composition as defined in any one of claims 1 to 3 which can be obtained by a process as defined in any one of claims 4 to 7.

9. Application of a composition according to any one of claims 1 to 3 and 8 or obtained according to the process described in any one of claims 4 to 7 as suspending agent, chickening agent and/or stabilizing agent for dispersions.

10. Application of a composition according to any one of claims 1 to 3 and 8 or obtained according to the process described in any one of claims 4 to 7 in the petroleum, agrochemical, food, cosmetics or explosives industries and in lubricating compositions or household or industrial cleaners.

11. Agrochemical, food, cosmetic, explosive, lubricating or household or industrial cleaning composition comprising a composition according to any one of claims 1 to 3 and 8 or obtained according to the process described in any one of claims 4 to 7.

## Patentansprüche

1. Zusammensetzung auf Basis von mindestens einem Succinoglykan-Derivat, dadurch gekennzeichnet, daß das ursprüngliche Succinoglykan ausgehend von dem Stamm *Agrobacterium tumefaciens I-736* erhalten worden ist und daß das Derivat einen um mindestens 30% verringerten Gehalt an von Brenztraubensäure abgeleiteten Motiven und einen um mindestens 50% (wobei 50% ausgeschlossen ist) verringerten Gehalt an von Bernsteinsäure abgeleiteten Motiven bezogen auf das ursprüngliche Succinoglykan aufweist und daß die Zusammensetzung sich in Form eines wäßrigen Gels oder eines Feststoffs vom Faser- oder Pulvertyp, der in wäßrigem Medium in ein Gel überführbar ist, befindet.

2. Zusammensetzung nach Anspruch 1, dadurch gekennzeichnet, daß bezogen auf das ursprüngliche Succinoglykan der Gehalt des Polysaccharids an Pyruvinmotiven um 30 bis 80% und an Succinmotiven um streng über 50% bis 80% verringert ist.

3. Zusammensetzung nach Anspruch 1, dadurch gekennzeichnet, daß die Konzentration des Gels an Polysaccharid zwischen 0,1 und 5% variiert.

4. Verfahren zur Herstellung einer Zusammensetzung, wie sie in einem der Ansprüche 1 bis 3 definiert ist, in Form eines wäßrigen Gels, dadurch gekennzeichnet, daß es umfaßt:
- Ansäuern einer wäßrigen Lösung eines Succinoglykans, das ausgehend von dem Stamm *Agrobacterium tumefaciens I-736* erhalten worden ist, mit einer wäßrigen Lösung einer Mineralsäure mit Ausnahme von Salpetersäure auf einen pH von mindestens unter 1,5,
- Inkubieren, gegebenenfalls unter Bewegung, dieser angesäuerten Lösung bei einer Temperatur über 40°C, bis diese eine minimale Viskositätsstufe erreicht und dann ein Gel bildet und
- Gewinnen der Zusammensetzung.

5. Verfahren nach Anspruch 4, dadurch gekennzeichnet, daß die Mineralsäure aus Salzsäure, Schwefelsäure und Phosphorsaure ausgewählt wird.

6. Verfahren nach einem der Ansprüche 4 oder 5, dadurch gekennzeichnet, daß die wäßrige Mineralsäurelösung eine 20%-ige Säurelösung ist.

7. Verfahren nach einem der Ansprüche 4 bis 6, dadurch gekennzeichnet, daß das Inkubieren der angesäuerten Lösung bei einer Temperatur zwischen 40 und 60°C erfolgt.

8. Zusammensetzung, wie sie in einem der Ansprüche 1 bis 3 definiert ist, die durch ein Verfahren, wie es in einem der Ansprüche 4 bis 7 definiert ist, erhalten werden kann.

9. Anwendung einer Zusammensetzung nach einem der Ansprüche 1 bis 3 und 8 oder erhalten gemäß dem in einem der Ansprüche 4 bis 7 beschriebenen Verfahren als Suspensionsmittel, Verdickungsmittel und/oder Dispersions-Stabilisierungsmittel.

10. Anwendung einer Zusammensetzung nach einem der Ansprüche 1 bis 3 und 8 oder erhalten gemäß dem in einem der Ansprüche 4 bis 7 beschriebenen Verfahren in der Erdölindustrie, agrochemischen Industrie, Lebensmittelindustrie, Kosmetikindustrie, Explosivstoffindustrie wie auch in Schmiermittelzusammensetzungen, Haushaltsreinigern oder industriellen Reinigungsmitteln.

11. Agrochemische Zusammensetzung, Lebensmittelzusammensetzung, kosmetische Zusammensetzung, explosive Zusammensetzung, Schmiermittelzusammensetzung, Haushaltsreiniger oder industrielles Reinigungsmittel, umfassend eine Zusammensetzung nach einem der Ansprüche 1 bis 3 und 8 oder erhalten gemäß dem in einem der Ansprüche 4 bis 7 beschriebenen Verfahren.
